(19) **Europäisches Patentamt / European Patent Office / Office européen des brevets**

(11) **EP 4 737 001 A1**

(12) # EUROPEAN PATENT APPLICATION
published in accordance with Art. 153(4) EPC

(43) Date of publication:
06.05.2026 Bulletin 2026/19

(21) Application number: 24830874.4

(22) Date of filing: 27.06.2024

(51) International Patent Classification (IPC):
$B01J\ 21/18^{(2006.01)}$    $B01J\ 35/60^{(2024.01)}$
$B01J\ 23/02^{(2006.01)}$    $B01J\ 21/04^{(2006.01)}$
$C07C\ 27/00^{(2006.01)}$    $C07C\ 29/128^{(2006.01)}$
$C07C\ 31/20^{(2006.01)}$    $C07C\ 68/065^{(2020.01)}$
$C07C\ 69/96^{(2006.01)}$

(52) Cooperative Patent Classification (CPC):
B01J 21/00; B01J 21/04; B01J 21/10; B01J 21/18;
B01J 23/02; B01J 23/04; B01J 27/04; B01J 27/24;
B01J 35/30; B01J 35/51; B01J 35/60; B01J 35/64;
C07C 27/00; C07C 29/128; C07C 31/20;    (Cont.)

(86) International application number:
PCT/CN2024/101940

(87) International publication number:
WO 2025/002241 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.06.2023 CN 202310769550

(71) Applicants:
• China Petroleum & Chemical Corporation
Beijing 100728 (CN)
• Sinopec (Shangai) Research Institute of
Petrochemical Technology Co., Ltd.
Shanghai 201208 (CN)

(72) Inventors:
• GE, Junwei
Shanghai 201208 (CN)
• YU, Fengping
Shanghai 201208 (CN)
• WANG, Yi
Shanghai 201208 (CN)
• HE, Wenjun
Shanghai 201208 (CN)

(74) Representative: karo IP
Patentanwälte PartG mbB
Steinstraße 16-18
40212 Düsseldorf (DE)

(54) **POROUS CARBON CARRIER-SUPPORTED CATALYST, PREPARATION METHOD THEREFOR AND USE THEREOF**

(57)    The present invention discloses a porous carbon support-loaded catalyst, a preparation process therefor, and an application thereof. In the catalyst of the present invention, calculated as active metal oxide, the content of the active metal component distributed within pore channels of the porous carbon support accounts for more than 50% relative to the total content of all active metal components in the catalyst. The catalyst of the present invention has stable properties in the system, and its active centers are not easily lost, solving the problems of easy loss of active components of the heterogeneous catalyst and short catalyst life existing in previous technologies. Additionally, when the catalyst of the present invention is used in the preparation of dialkyl carbonates, it exhibits excellent conversion rate and selectivity.

Fig. 1

**(Cont. next page)**

(52) Cooperative Patent Classification (CPC): (Cont.)
    **C07C 68/065; C07C 69/96**

**Description**

Technical Field

The present invention provides a supported catalyst, and more specifically relates to a catalyst in which an active metal component is mainly loaded within the pore channels of a porous carbon support, a preparation method for said catalyst, and an application thereof in the preparation of dialkyl carbonates.

Background Technology

Dimethyl carbonate (DMC), characterized by its active chemical properties, excellent physical properties, non-toxicity, and ease of biodegradation, is a new, low-pollution, environmentally friendly green basic chemical raw material. It can be used as a solvent, a gasoline additive, a lithium-ion battery electrolyte, and a carbonylation, methylation, or carbomethoxylation reagent, and is widely applied in the chemical and chemical engineering fields. Currently, countries worldwide are actively researching green chemical processes based on this environmentally friendly chemical raw material, DMC. Among them, the transesterification method is superior due to its mild reaction conditions, high yield, and co-production of ethylene glycol or propylene glycol, making it a highly promising industrial approach.

Generally, alkali metal hydroxides, alkali metal carbonates, alkali metal alkoxides, etc. are used as catalysts in transesterification reactions (US2011040117A1; WO2010063780A1). However, because they are homogeneous catalysts, they are difficult to separate from the products, leading to challenges in reuse. Commonly used heterogeneous catalysts include alkali metals or alkali metal salts supported on supports, metal oxide catalysts, alkali (earth) metal-exchanged zeolites or clay materials, ion exchange resins and the like. Alkali metals or alkali metal salts supported on supports, such as $KF/Al_2O_3$, NaOH/chitosan, and $Cs_2CO_3/SiO_2-Al_2O_3$ (CN101249452A; CN101121147A; WO2001056971A1), have the disadvantage of being easily affected by water and CO2 in the air and easily lost during the reaction, resulting in decreased activity. Metal oxide catalysts, such as $Al_2O_3$ and MgO (US2005080287A1; US6207850B1), and alkali (earth) metal-exchanged zeolites or clay materials, such as Cs-ZSM-5 and Mg-smectite (WO2000073256A1), have the disadvantage of generally low activity or selectivity. Ion exchange resins, such as quaternary ammonium-type or tertiary amine-type resins, are usually not resistant to high temperatures or swelling, and their activity decreases rapidly over time.

Therefore, finding a catalyst with high stability and high conversion rate for the preparation of dialkyl carbonates remains an urgent need in this field.

Summary of the Invention

In view of the above situation, the inventors of the present invention conducted in-depth research and surprisingly found that by loading an active metal component onto a modified resin support and then calcining the resin support, a catalyst in which the active metal component is mainly distributed within the pore channels of a porous carbon support can be obtained. This catalyst exhibits excellent stability and high catalytic activity, thereby completing the present invention.

Specifically, a first aspect of the present invention provides a porous carbon support-loaded catalyst, characterized in that it comprises a porous carbon support, an active metal component, and at least one heteroatom selected from nitrogen, sulfur, and phosphorus, wherein, relative to the total mass of the catalyst, the content of the porous carbon support is 50% to 99%, the content of the active metal component calculated as active metal oxide is 0.1% to 50%, and the content of the heteroatom(s) calculated as element is 0.1% to 5%; said active metal is at least one selected from magnesium, aluminum, and calcium; and calculated as active metal oxide, the content of the active metal component distributed within porous channels of the porous carbon support accounts for more than 50% relative to the total content of all active metal components in the catalyst.

A second aspect of the present invention provides a process for preparing a catalyst, comprising the following steps:

(1) a modification step, in which a haloalkylated resin is contacted with a modifier to perform a modification, thereby obtaining a modified haloalkylated resin;
(2) a contact step, in which the modified haloalkylated resin is contacted with a metal precursor of an active metal, thereby obtaining a contact product; and
(3) a calcining step, in which the contact product is optionally dried and then calcined in an inactive gas atmosphere.

A third aspect of the present invention provides a method for preparing a dialkyl carbonate, wherein a carbonate and a lower alcohol are reacted in the presence of the aforementioned catalyst of the present invention or a catalyst prepared by the preparation process of the present invention to obtain the dialkyl carbonate.

Technical effect

In the catalyst of the present invention, compared to the active metal component distributed on the surface of the porous carbon support, the content of the active metal component that enters and is distributed within the pore channels of the porous carbon support is higher, accounting for more than 50% relative to the total content of all active metal components contained in the catalyst. Consequently, the catalyst of the present invention exhibits excellent conversion rate and selectivity in the preparation of dialkyl carbonates, has a long catalyst life, and is suitable for industrial use. Moreover, when this carbon support-load catalyst is used in the preparation of dialkyl carbonates, the catalyst system has stable properties, and the active centers are not easily lost, solving the problems of easy loss of active components of the heterogeneous catalyst and short catalyst life existing in previous technologies.

In the preparation process of the catalyst of the present invention, by using a spherical porous resin support, the utilization rate of the support and the density of active centers can be effectively improved, solving the problem of low activity of heterogeneous catalysts in the prior art. By modifying the resin, subsequently loading the precursor of the active metal, and then calcining, the active metal component is enabled to enter the pore channels of the carbon support to a greater extent, thereby efficiently preparing the catalyst of the present invention.

In the method for preparing a dialkyl carbonate of the present invention, by using the catalyst of the present invention or a catalyst prepared by the preparation process of the present invention, a dialkyl carbonate can be prepared from a carbonate and a lower alcohol with a high conversion rate and high selectivity of dialkyl carbonate.

Brief description of the drawings

Figure 1 shows a scanning electron micrograph of catalyst C1 prepared in Example 1 of the present invention.
Figure 2 shows a scanning electron micrograph of catalyst S1 prepared in Comparative Example 1 of the present invention.
Figure 3 shows a transmission electron micrograph of catalyst C1 prepared in Example 1 of the present invention.
Figure 4 shows a transmission electron micrograph of catalyst S1 prepared in Comparative Example 1 of the present invention.
Figure 5 shows (a) a scanning electron micrograph of catalyst C1 prepared in Example 1 of the present invention at a 100-micron scale; (b) a scanning electron micrograph of the interior of the catalyst prepared in Example 1 of the present invention at a 10-micron scale.
Figure 6 shows (a) a scanning electron micrograph of the interior of catalyst C1 prepared in Example 1 of the present invention at a 200-nanometer scale; (b) a scanning electron micrograph of the interior of catalyst C1 prepared in Example 1 of the present invention at a 100-nanometer scale.
Figure 7 shows an Energy Dispersive Spectroscopy mapping and EDX spectrum of catalyst C1 prepared in Example 1 of the present invention; (a) selected area mapping; (b) carbon element distribution map; (c) oxygen element distribution map; (d) magnesium element distribution map; (e) EDX energy spectrum.
Figure 8 shows (a) a scanning electron micrograph of catalyst S1 prepared in Comparative Example 1 of the present invention at a 100-micron scale; (b) a scanning electron micrograph of the interior of the catalyst S1 prepared in Comparative Example 1 of the present invention at a 20-micron scale.
Figure 9 shows (a) a scanning electron micrograph of catalyst S1 prepared in Comparative Example 1 of the present invention at a 200-nanometer scale; (b) a scanning electron micrograph of the interior of the catalyst S1 prepared in Comparative Example 1 of the present invention at a 100-nanometer scale.
Figure 10 shows an Energy Dispersive Spectroscopy mapping and EDX spectrum of catalyst S1 prepared in Comparative Example 1 of the present invention; (a) selected area mapping; (b) carbon element distribution map; (c) EDX energy spectrum.

Detailed description

The present invention is described in detail below in conjunction with specific embodiments, but it should be noted that the scope of the present invention is not limited by these specific embodiments and principled explanations, but is defined by the claims. In the present invention, except for what is explicitly indicated, any item or matter not mentioned is directly applicable to those known in the art without any changes. Moreover, any of the embodiments described herein can be freely combined with one or more other embodiments described herein, and the resulting technical solutions or technical ideas are regarded as part of the original disclosure or the original record of the present invention, and should not be regarded as new content that has not been disclosed or anticipated in this specification, unless those skilled in the art believe that the combination is obviously unreasonable.

All features disclosed in the present invention can be arbitrarily combined. These combinations should be understood as the content disclosed or recorded by the present invention. Unless those skilled in the art consider the combination obviously unreasonable, they should be deemed as specifically disclosed and recorded by the present invention. The numerical points disclosed in this specification, unless specifically indicated, include not only the specific numerical points disclosed in the examples, but also the endpoints of the numerical ranges in the specification. Any range formed by the arbitrary combination of these numerical points should be considered as the range disclosed or recorded by the present invention.

The technical and scientific terms in the present invention shall be understood according to their definitions if defined herein, and according to the common meanings in the art if not defined herein.

[Carbon Support-Load Catalyst]

The present invention provides a porous carbon support-loaded catalyst, which comprises a porous carbon support, an active metal component, and at least one heteroatom selected from nitrogen, sulfur, and phosphorus, wherein, relative to the total mass of the catalyst, the content of the porous carbon support is 50% to 99%, the content of the active metal component calculated as active metal oxide is 0.1% to 50%, and the content of the heteroatom(s) calculated as element is 0.1% to 5%; said active metal is at least one selected from magnesium, aluminum, and calcium; and calculated as active metal oxide, the content of the active metal component distributed within porous channels of the porous carbon support accounts for more than 50% relative to the total content of all active metal components in the catalyst.

In the catalyst of the present invention, the porous carbon support is derived from the calcination of a porous resin material in an inert gas.

In the catalyst of the present invention, the particle size of the porous carbon support is not particularly limited, and can be 0.1 mm to 3 mm, preferably 0.3 mm to 2 mm. The pore size of the porous carbon support of the present invention is not particularly limited, and can be, for example, 5 to 500 nm, preferably 50 to 300 nm.

In one embodiment of the present invention, relative to the total mass of the catalyst, the content of the porous carbon support is 50% to 99%, preferably 55% to 90%, more preferably 60% to 85%.

In the present invention, the at least one heteroatom selected from nitrogen, sulfur, and phosphorus is derived from the modifier used in preparing the catalyst. During the calcining step, the modifier is calcined, leaving behind at least one heteroatom selected from nitrogen, sulfur, and phosphorus.

In one embodiment of the present invention, relative to the total mass of the catalyst, the content of the at least one heteroatom selected from nitrogen, sulfur, and phosphorus calculated as element is 0.1% to 5%; preferably 0.3% to 4.5%, more preferably 0.5% to 4%.

In the present invention, the catalyst also contains an oxygen element; however, the present invention does not intend to limit the content of the oxygen element. Generally, the content of the oxygen element is included in the oxides of the present invention.

In one embodiment of the present invention, the catalyst may also contain a halogen element derived from the haloalkylated resin. Usually, during calcining, the halogen element is removed by calcining, but an impurity level of the halogen element may still remain. The present invention does not intend to limit the content of the halogen element.

In the present invention, the content of the active metal component (calculated as oxide), the content of the heteroatom(s) (calculated as element), and the content of the optional halogen atom(s) (calculated as element) in the catalyst can be determined using methods known in the art, such as elemental analysis. After deducting the content of the active metal oxide, the content of the heteroatom(s), and the content of the optional halogen atom(s), the remaining content is recorded as the content of the porous carbon support of the present invention. In the present invention, the active metal oxide is derived from the calcining product of the active metal precursor introduced during the catalyst preparation.

In one embodiment of the present invention, relative to the total mass of the catalyst, the content of the active metal component calculated as active metal oxide is 0.1% to 50%, preferably 5% to 45%, more preferably 10% to 40%.

In one embodiment of the present invention, the active metal is at least one selected from magnesium, aluminum, and calcium, preferably at least one selected from magnesium and calcium.

In the catalyst of the present invention, the active metal component includes the active metal in any valence state and/or in any oxidation state. Typically, in the catalyst of the present invention, the active metal component usually exists in the form of a metal oxide, which is a product after calcining the precursor of the active metal.

In the present invention, unless otherwise specified, the active metal component is usually measured in the form of the active metal oxide.

In the catalyst of the present invention, compared to the active metal component distributed on the surface of the porous carbon support (sometimes also referred to herein as the outer surface or exterior), the active metal component distributed within the pore channels of the porous carbon support (sometimes also referred to herein as the inner surface or interior) has a higher content.

In one embodiment of the present invention, calculated as active metal oxide, the content of the active metal component distributed within pore channels of the porous carbon support accounts for more than 50%, preferably 55% or more, more preferably 60% or more, further preferably 65% or more, most preferably 75% or more relative to the total content of all active metal components in the catalyst. The upper limit for the content of the active metal component distributed within pore channels of the porous carbon support relative to the total content of all active metal components in the catalyst is not particularly limited, preferably 100%, but from a practical production cost perspective, the upper limit can be 99%, 98%, 97%, 96%, or 95%.

In one embodiment of the present invention, calculated as active metal oxide, the content of the active metal component distributed on the surface of the porous carbon support (outer surface or exterior) accounts for less than 50%, preferably 45% or less, more preferably 40% or less, further preferably 35% or less, most preferably 30% or less, relative to the total content of all active metal components in the catalyst. The lower limit for the content of the active metal component distributed on the surface (outer surface or exterior) relative to the total content of all active metal components in the catalyst is as low as possible, preferably 0, but from a practical production cost perspective, the lower limit can be 5%, 4%, 3%, 2%, or 1%.

In the present invention, the content of the active metal component distributed on the surface of the porous carbon support (outer surface or exterior) can be determined by XPS. That is, in the present invention, the content of the active metal of the porous carbon support measured by XPS is taken as the content of the active metal component on the surface of the porous carbon support (outer surface or exterior).

It should be noted that in the present invention, the content of the active metal component within the pore channels of the porous carbon support (inner surface or interior) can be obtained by subtracting the content of the active metal component distributed on the surface of the porous carbon support (outer surface or exterior) from the total content of all active metal components in the porous carbon support. Here, the total content of all active metal components in the catalyst can be determined by elemental analysis and the like; on the other hand, in the present invention, the content of the active metal component of the porous carbon support measured by XPS is taken as the content of the active metal component distributed on the surface of the porous carbon support (outer surface or exterior). Thus, based on these two, the content of the active metal component distributed within the pore channels of the porous carbon support (internal surface or interior) can be calculated: the content of the active metal component distributed within the pore channels (internal surface or interior)="the total content of all active metal components in the porous carbon support" minus "the content of the active metal component distributed on the surface (outer surface or exterior) of the porous carbon support measured by XPS".

In the present invention, by allowing a majority (e.g., over 50%) of the active metal component to be distributed within the pore channels of the porous carbon support, more preferably by allowing a greater majority (e.g., 55% or more, preferably 60% or more, further preferably 65% or more, most preferably 70% or more) of the active metal component to be distributed within the pore channels of the porous carbon support, the catalyst exhibits excellent conversion rate, high selectivity, and a greatly extended catalyst life.

In one embodiment of the present invention there is provided a catalyst for preparing dimethyl carbonate, comprising a support and an active component, characterized in that the mass percentages of the support and the active component in the catalyst are: support 50% to 99.9%, active component 0.1% to 50%; said support is carbon, said active component is at least one selected from magnesium, calcium, and aluminum; said carbon is obtained by the carbonization of a modified chloromethylated resin.

The active component is distributed on the surface of the support and within the pore channels of the support, and the content of the active component within the pore channels of the support is greater than the content of the active component on the surface of the support.

[Process for Preparing the Catalyst]

A second aspect of the present invention provides a process for preparing a catalyst, comprising the following steps:

(1) a modification step, in which a haloalkylated resin is contacted with a modifier to perform a modification, thereby obtaining a modified haloalkylated resin;
(2) a contact step, in which the modified haloalkylated resin is contacted with a metal precursor of an active metal, thereby obtaining a contact product; and
(3) a calcining step, in which the contact product is optionally dried and then calcined in an inactive gas atmosphere,

said modifier is at least one compound selected from nitrogen-containing compounds, sulfur-containing compounds, and phosphorus-containing compounds.

The inventors of the present invention have found that by modifying a haloalkylated resin with a modifier, allowing the modified resin to interact with an active metal precursor, and then calcining, a catalyst can be obtained in which the active metal component is mainly distributed within the pore channels of the porous carbon support. In the present invention, halo or halogen can be exemplified by fluorine, chlorine, bromine, and iodine, preferably chlorine or iodine, more preferably chlorine.

In the present invention, the alkyl group in the alkylation can be exemplified by $C_1$-$C_6$alkyl, preferably $C_1$-$C_4$alkyl, more preferably methyl, ethyl, propyl, or isopropyl. In one embodiment of the present invention, the haloalkylation is preferably halomethylation, more preferably chloromethylation.

In the present invention, the haloalkylated resin can be obtained by a haloalkylation modification (preferably halomethylation modification, more preferably chloromethylation modification) of a conventional resin, or a commercially available haloalkylated resin can be used, as long as the resin has a porous structure.

In the present invention, the haloalkylated resin can be exemplified by haloalkylated styrene resin, e.g. chloromethylated resin, particularly chloromethylated styrene resin. As the haloalkylated styrene resin, it can be a commercially available product, or can be prepared by methods known in the art, including but not limited to the following methods: alkylating a poly-para-styrene with a haloalkene ($C_2$-$C_6$haloalkene) to produce a haloalkylated polystyrene (CN1137788A). In addition, as the chloromethylated styrene resin, it can be a commercially available product, or can be prepared by methods known in the art, including but not limited to the following methods: preparing a polystyrene microsphere resin by suspension polymerization, and reacting with chloromethyl ether to obtain a chloromethylated styrene resin (CN103864973A). In addition, methods disclosed in CN103709278A, CN104788592A, and the like can be referred to for preparing the haloalkylated styrene resin or the chloromethylated styrene resin.

In one embodiment of the present invention, the particle size of the haloalkylated resin is 0.1 mm to 3 mm, preferably 0.3 mm to 2 mm.

In one embodiment of the present invention, the pore size of the haloalkylated resin is 5 to 500 nm, preferably 50 to 300 nm.

In one embodiment of the present invention, the haloalkylated resin is spherical.

In step (1) of the present invention, the modifier is contacted with the haloalkylated resin for modification to obtain a modified haloalkylated resin (hereinafter sometimes also referred to as "modification product" or "modified resin"). The present invention does not intend to provide a restrictive explanation of the reaction occurring at this time; it is speculated that it may be due to the interaction of the heteroatoms (nitrogen, phosphorus, or sulfur) contained in the modifier with the haloalkyl group. Moreover, the introduced heteroatoms can promote the subsequent dispersion of the active metal precursor in the support, improve the dispersion of the metal active component, promote the alkalinity, and cause the active metal component to deposit within the pore channels of the porous carbon support after calcining.

Therefore, in the present invention, the modifier is at least one compound selected from nitrogen-containing compounds, sulfur-containing compounds, and phosphorus-containing compounds.

In one embodiment of the present invention, the nitrogen containing compound is a nitrogen-containing organic compound.

In one embodiment of the present invention, the nitrogen-containing organic compound can be any organic compound containing nitrogen atom(s) without particular limitation, and alkylamines or 5- or 6-membered nitrogen-containing heterocyclic aromatic compound can be used. Examples of alkylamines may include methylamine, ethylamine, propylamine, butylamine, pentylamine, hexylamine, cyclohexylamine, octylamine, decylamine, dimethylamine, diethylamine, dibutylamine, trimethylamine, triethylamine, tributylamine, etc. Examples of 5- or 6-membered nitrogen-containing heterocyclic aromatic compounds may include at least one of 5- or 6-membered nitrogen-containing heterocyclic aromatic compounds optionally substituted by $C_1$-$C_6$alkyl. Examples of 5- or 6-membered nitrogen-containing heterocyclic aromatic compounds may include piperazine, pyridine, imidazole, 1-methylimidazole, quinoline, pyrrole, indole, carbazole, isoindole, and thiazole. These nitrogen-containing heterocyclic aromatic compounds are optionally substituted by $C_1$-$C_6$alkyl. In the present invention, the $C_1$-$C_6$alkyl is preferably $C_1$-$C_4$alkyl, more preferably methyl, ethyl, propyl, or isopropyl. In one embodiment of the present invention, the number of substituents for "substituted by $C_1$-$C_6$alkyl" can be 1, 2, 3, 4, or 5, preferably 1, 2, or 3, more preferably 1 or 2.

In one embodiment of the present invention, the sulfur-containing compound is a sulfur-containing organic compound.

In one embodiment of the present invention, the sulfur-containing organic compound can be any organic compound containing sulfur atom(s) without particular limitation, and for example, can be exemplified by thioethers such as dimethyl thioether, diethyl thioether, dipropyl thioether, dihexyl thioether, dicyclohexyl thioether and diphenyl thioether; dialkyl disulfide compounds such as dimethyl disulfide, diethyl disulfide, dipropyl disulfide, dibutyl disulfide, dihexyl disulfide, dicyclohexyl disulfide and ethyl methyl disulfide; thiophenes optionally substituted by $C_1$-$C_6$alkyl such as thiophene, 2-methylthiophene, 3-methylthiophene, 2,3-dimethylthiophene, 2-ethylthiophene and benzothiophene; heterocyclic sulfur-containing compounds such as tetrahydrothiophene and thiopyrane; aromatic sulfur-containing compounds such as diphenyl sulfide, diphenyl disulfide, methylphenyl disulfide and methylphenyl sulfide: thiourea; and sulfides such as methyl sulfide, ethyl sulfide, and butyl sulfide and the like.

In one embodiment of the present invention, the phosphorus-containing compound is a phosphorus-containing organic compound.

In one embodiment of the present invention, the phosphorus-containing organic compound can be any organic compound containing phosphorus atom(s) without particular limitation, and for example can be exemplified by phosphines such as triphenylphosphine, triethylphosphine, tributylphosphine, tripropylphosphine, trioctylphosphine, and tricyclohexylphosphine.

In one embodiment of the present invention, the modifier is preferably at least one selected from imidazole optionally substituted by $C_1$-$C_6$alkyl, pyridine optionally substituted by $C_1$-$C_6$alkyl, thiophene optionally substituted by $C_1$-$C_6$alkyl, thiazole optionally substituted by $C_1$-$C_6$alkyl, piperazine optionally substituted by $C_1$-$C_6$alkyl and triphenylphosphine, more preferably at least one selected from imidazole, 1-methylimidazole, pyridine, piperazine and thiophene.

In one embodiment of the present invention, the mass ratio of the haloalkylated resin to the modifier is 1:(0.1-1.2), preferably 1:(0.15-1), for example, 1:1, 1:0.8, or 1:0.6.

In one embodiment of the present invention, the modification step can be carried out in a solvent. As the solvent, it can be at least one selected from toluene, methanol, ethanol, n-propanol, n-butanol, isobutanol, tert-butanol, tetrahydrofuran, dichloromethane, trichloromethane, N,N-dimethylformamide, ether, and water.

In one embodiment of the present invention, the mass ratio of the haloalkylated resin to the solvent is 1:(1-10), preferably 1:(2-8), for example, 1:3.792, or 1:5.688.

In one embodiment of the present invention, the modification step can be carried out at a temperature of 30°C to 150°C, preferably 40°C to 120°C, for example, 70°C, 80°C, or 90°C. The solution containing the resin raw material can be heated before the contact of the resin raw material with the modifier, or the solution can be heated during the contact of the resin raw material with the modifier.

In the present invention, the modification time for the modification step is not particularly limited. In one embodiment of the present invention, the modification time for the modification step is 1 h to 24 h, preferably 2 h to 18 h, for example, 12 h, or 15 h.

In one embodiment of the present invention, during the modification, mixing and stirring of the solution containing the resin raw material and the modifier can also be performed. The mixing and stirring rate is 50 rpm to 1000 rpm, preferably 100 rpm to 800 rpm, for example, 500 rpm, or 800 rpm. The stirring time is not particularly limited, and can be, for example, 2 h.

In one embodiment of the present invention, in step (1), the obtained modification product can also be washed and dried as needed. At this time, the aforementioned solvent can be used for washing, followed by drying. As the drying condition, the drying temperature can be 80°C to 180°C, for example, 100°C, or 120°C; the drying time can be 1 h to 48 h, for example, 24 h.

In step (2) of the present invention, the modified haloalkylated resin (modification product) is contacted with a metal precursor of an active metal to obtain a contact product.

In the present invention, in step (2), the metal precursor of the active metal is loaded onto the modified haloalkylated resin.

In the present invention, the active metal is at least one selected from magnesium, calcium, and aluminum, preferably at least one selected from magnesium and calcium. In one embodiment of the present invention, the metal precursor of the active metal is in at least one form of nitrate, chloride, and sulfate of the active metal.

In one embodiment of the present invention, the mass ratio of the modified haloalkylated resin to the metal precursor of the active metal is 1:0.1 to 3, preferably 1:0.2 to 2.

In one embodiment of the present invention, the metal precursor of the active metal exists in the form of solution. In this case, the solvent can be selected from the aforementioned solvents, preferably water. In one embodiment of the present invention, the mass concentration of the solution of the metal precursor of the active metal is 5% to 50%, for example, 6.5%, 7%, 8%, 8.8%, 17.6%, 26.4%, or 35.2%.

In one embodiment of the present invention, the mass ratio of the modified haloalkylated resin to the solution containing the metal precursor of the active metal is 1:(1-10), preferably 1:(2-6).

In one embodiment of the present invention, the contact step is carried out in the presence of a solvent. As the solvent, it can be at least one selected from toluene, methanol, ethanol, n-propanol, n-butanol, isobutanol, tert-butanol, tetrahydrofuran, dichloromethane, trichloromethane, N,N-dimethylformamide, ether, and water.

In one embodiment of the present invention, the solution containing the modified resin and the metal precursor of the active metal are mixed and stirred. The conditions for mixing and stirring are not particularly limited. For example, the mixing and stirring rate is 100 rpm to 800 rpm, preferably 200 rpm to 600 rpm; the stirring temperature is 30°C to 100°C, preferably 50°C to 80°C, for example, 50°C, or 80°C; the stirring time is 1 h to 10 h, preferably 2 h to 8 h, for example, 2 h, 6 h, or 8 h.

In step (3) of the present invention, the product obtained from the contact step is calcined in an inactive gas atmosphere to obtain the catalyst of the present invention.

In one embodiment of the present invention, in step (3) of the present invention, the product obtained from the contact step is first dried and then calcined. The drying conditions are not particularly limited, for example, the temperature is 80°C to 180°C, preferably 90°C to 150°C, for example, 100°C; and the time is 1 h to 48 h, preferably 2 h to 36 h, for example, 14 h, or 24 h.

In the present invention, the modified resin particles loaded with the metal precursor of the active metal are calcined. During calcining, the resin particles undergo carbonization and transform into a porous carbon support, which serves as the porous carbon support of the catalyst of the present invention and on which the active metal component is mainly enriched within the pore channels of the porous carbon support (inner surface or interior), and at the same time, the modifier is removed, leaving behind at least one heteroatom selected from nitrogen, sulfur, and phosphorus in the carbon support.

In the present invention, the inactive gas is not limited, as long as it is an inactive gas known in the art. In one embodiment of the present invention, the inactive gas (inert gas) can be exemplified by nitrogen, and rare gases such as argon.

In one embodiment of the present invention, the calcining temperature is 200°C to 1000°C, preferably 250°C to 600°C, for example, 400°C, 500°C, or 600°C; and the calcining time is 1 h to 48 h, preferably 2 h to 24 h, for example, 4 h, 5 h, or 8 h.

One embodiment of the present invention provides a process for preparing a catalyst for preparing dimethyl carbonate, wherein when preparing the modified chloromethylated resin, a chloromethylated resin is mixed with a solvent, a modifier is added, and the modification is performed to obtain the modified chloromethylated resin; preferably, the modifier is a nitrogen-containing 5- or 6-membered heterocyclic aromatic compound, the modified chloromethylated resin is a modified chloromethylated styrene resin, the solvent is at least one selected from toluene, methanol, ethanol, n-propanol, n-butanol, isobutanol, tert-butanol, tetrahydrofuran, dichloromethane, trichloromethane, N,N-dimethylformamide, ether, and water; further preferably, the chloromethylated resin is styrene-based, the modifier is at least one selected from piperazine, imidazole, and pyridine; still further preferably, the particle size of the chloromethylated resin is 0.1 mm to 3 mm, preferably 0.3 mm to 2 mm; the chloromethylated resin is spherical;

and/or, the mass ratio of the chloromethylated resin to the solvent is 1:(1 to 10), preferably 1:(2 to 8);

and/or, the mass ratio of the chloromethylated resin to the modifier is 1:(0.1-1.2), preferably 1:(0.15-1);

and/or, before adding the modifier, a heating step is also included; the temperature after heating is 30°C to 150°C, preferably 40°C to 120°C;

and/or, the mixing and stirring rate is 50 rpm to 1000 rpm, preferably 100 rpm to 800 rpm; the mixing and stirring time is 2 h;

and/or, the preparation process comprises a step of loading an active component onto the support.

In one embodiment of the present invention, when loading the active component onto the support, the support and a solution containing the metal precursor of the active component are mixed and stirred, dried, and then calcined in an inactive gas atmosphere to obtain the catalyst.

In one embodiment of the present invention, the solution containing the metal precursor of the active component is at least one selected from a nitrate, chloride, or sulfate solution of magnesium, calcium, or aluminum, and the mass concentration of the solution containing the metal precursor of the active component is 5% to 50%.

In one embodiment of the present invention, when loading the active component onto the support, the mass ratio of the support to the solution containing the metal precursor of the active component is 1:(1 to 10), preferably 1:(2 to 6);

and/or, the stirring rate is 100 rpm to 800 rpm, the stirring temperature is 50°C to 80°C, and the stirring time is 2 h to 8 h;

and/or, the drying temperature is 80°C to 180°C, and the time is 1 h to 48 h;

and/or, the inactive gas is nitrogen or argon;

and/or, the calcining temperature is 200°C to 1000°C, and the time is 1 h to 48 h; preferably, the calcining temperature is 250°C to 600°C, and the time is 2 h to 24 h.

[Preparation Method for Dialkyl Carbonates]

A third aspect of the present invention provides a preparation method for a dialkyl carbonate represented by the following formula (I), comprising a step of reacting a carbonate represented by the following formula (II) and a lower alcohol represented by the following formula (III) to obtain the dialkyl carbonate represented by formula (I); the catalyst being the aforementioned catalyst of the present invention or a catalyst prepared by the aforementioned preparation method of the present invention,

$$R_1\text{-OCOO-}R_1 \qquad (I)$$

in formula (I), each $R_1$ is independently selected from $C_1$-$C_6$alkyl;

$$R_2\text{-OCOO-}R_2 \qquad (II)$$

in formula (II), each $R_2$ is independently selected from $C_1$-$C_6$alkyl, and at least one $R_2$ is different from at least one $R_1$; or the two $R_2$ are bonded to each other to form a $C_2$-$C_6$ alkylene group;

$$R_1\text{-OH} \qquad (III)$$

in formula (III), $R_1$ is selected from $C_1$-$C_6$alkyl.

In one embodiment of the present invention, the reaction temperature for preparing the dialkyl carbonate represented by formula (I) is 40°C to 130°C, preferably 50°C to 120°C, for example, 70°C, 80°C, 90°C, 110°C, or 120°C.

In one embodiment of the present invention, the reaction time for preparing the dialkyl carbonate represented by formula (I) is 1 h to 15 h, preferably 2 h to 12 h, for example, 4 h, 6 h, 8 h, or 10 h.

In one embodiment of the present invention, in the preparation of the dialkyl carbonate represented by formula (I), the molar ratio of the lower alcohol represented by formula (III) to the carbonate represented by formula (II) is (0.5-15):1, preferably (1-12):1, for example, 4:1, 6:1, or 8:1.

In one embodiment of the present invention, in the preparation of the dialkyl carbonate represented by formula (I), the mass ratio of the catalyst to the carbonate represented by formula (II) is (0.001-0.5):1, preferably (0.05-0.5):1, for example, 0.2:1, 0.3:1, or 0.4:1.

In one embodiment of the present invention, in the dialkyl carbonate represented by formula (I), the two $R_1$ may be identical or different. When the two $R_1$ are identical, the dialkyl carbonate is a symmetric dialkyl carbonate; when the two $R_1$ are different, the dialkyl carbonate is an asymmetric dialkyl carbonate. In the present invention, $R_1$ can be exemplified by methyl, ethyl, propyl, isopropyl, butyl, isobutyl, tert-butyl, and sec-butyl. Therefore, in the present invention, the dialkyl carbonate represented by formula (I) can be exemplified by dimethyl carbonate, diethyl carbonate, ethyl methyl carbonate, dipropyl carbonate, dibutyl carbonate, etc.

In one embodiment of the present invention, in the carbonate represented by formula (II), each $R_2$ is independently selected from $C_1$-$C_6$alkyl, and at least one $R_2$ is different from at least one $R_1$. In the present invention, $R_2$ can be exemplified by methyl, ethyl, propyl, isopropyl, butyl, isobutyl, tert-butyl, sec-butyl, pentyl, and hexyl.

In one embodiment of the present invention, in the carbonate represented by formula (II), the two $R_2$ are bonded to each other to form a $C_2$-$C_6$ alkylene group. In this case, the carbonate represented by formula (II) is a cyclic carbonate, for example, ethylene carbonate, and propylene carbonate.

In the present invention, one or two or more kinds of the lower alcohols represented by formula (III) can be used. As the lower alcohol of the present invention, methanol, ethanol, propanol, isopropanol, butanol and isomers thereof can be exemplified.

In the present invention, $R_1$ in the lower alcohol represented by formula (III) is identical to at least one of the two $R_1$ in the dialkyl carbonate represented by formula (I).

In the present invention, $R_2$ and $R_1$ may be identical, but at least one $R_2$ is different from at least one $R_1$.

In other words, in the present invention, the carbonate represented by formula (II) and the lower alcohol represented by formula (III) are subjected to a transesterification reaction, causing the $R_1$ from the lower alcohol represented by formula (III) to become at least one terminal of the dialkyl carbonate represented by formula (I).

One embodiment of the present invention provides a method for preparing dimethyl carbonate, characterized in that it comprises a step of reacting an alkylene carbonate and methanol in the presence of a catalyst to obtain dimethyl carbonate; the catalyst being the catalyst of the present invention;

and/or, the reaction temperature is 40°C to 130°C, preferably 50°C to 120°C;
and/or, the reaction time is 1 h to 15 h, preferably 2 h to 12 h,
and/or, the molar ratio of the methanol to the alkylene carbonate is (2-15):1, preferably (3-12):1;
and/or, the mass ratio of the catalyst to the alkylene carbonate is (0.001-0.5):1, preferably (0.05-0.5):1;
and/or, the alkylene carbonate is at least one selected from ethylene carbonate and propylene carbonate.

When the catalyst of the present invention or a catalyst prepared by the aforementioned preparation process of the present invention is used in the transesterification reaction of a carbonate represented by formula (II) and a lower alcohol represented by formula (III) to prepare dialkyl carbonates, even after multiple (e.g., more than 10) reuses, the decrease of its activity is less than 5%, achieving a good technical effect.

As an example of the condition for preparing the dialkyl carbonate, the following can be exemplified: the reaction temperature is 80°C, the molar ratio of lower alcohol to cyclic carbonate is 8:1, and the mass ratio of catalyst to cyclic carbonate is 0.3:1, and the reaction time is 10 h. In this case, the conversion rate of the cyclic carbonate is 80.4%, and the selectivity for dialkyl carbonate is 99.9%.

Examples

The present invention is further described below in combination with examples, but the present invention is not limited by these examples.

In the present invention, the gas chromatograph was an Agilent 7890 chromatograph. Analysis conditions were: Chromatographic column HP-FFAP, injection port temperature 280°C, FID detector, column oven temperature programming, maintained at 50°C for 3 minutes, then increased at 10°C/min to 230°C and held for 10 minutes, split ratio 50:1, component content analysis by calibration normalization method. Heteroatom content elemental analysis was performed using a Therm2000 instrument. XPS testing was performed on an X-ray photoelectron spectrometer (XPS, AXIS ULTRA DLD) instrument from Kratos Company, Japan.

Metal element analysis testing was performed by ICP-OES, using a Varian 725ES instrument.

SEM was performed using a scanning electron microscope (SEM, Merlin) from ZEISS Company, Germany.

TEM was obtained by using a Tecnai 20 S-TWIN transmission electron microscope under an operation voltage of 200 kV.

In the examples, the calculation method for the conversion rate of raw material was:

(Mass of input raw material-Mass of product after reaction*Raw material content)/Mass of input raw material* 100%.

In the examples, the calculation method for the selectivity of carbonate is: Yield of carbonate product after reaction/Theoretical carbonate yield from consumed raw material*100%.

The calculation method for the proportion of the active metal component distributed within the pore channels of the porous carbon support is: (the content of the active metal component in the catalyst calculated as active metal oxide-the content of the active metal component calculated as active metal oxide detected by XPS)/the content of the active metal component in the catalyst calculated as active metal oxide.

Example 1

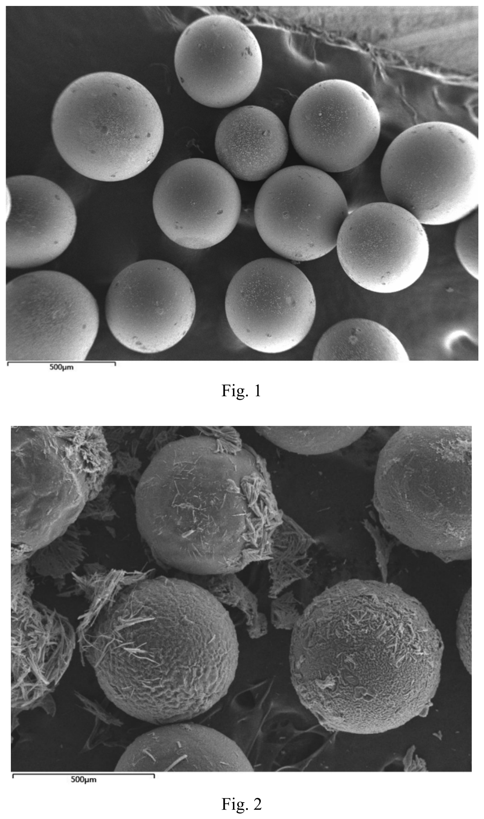

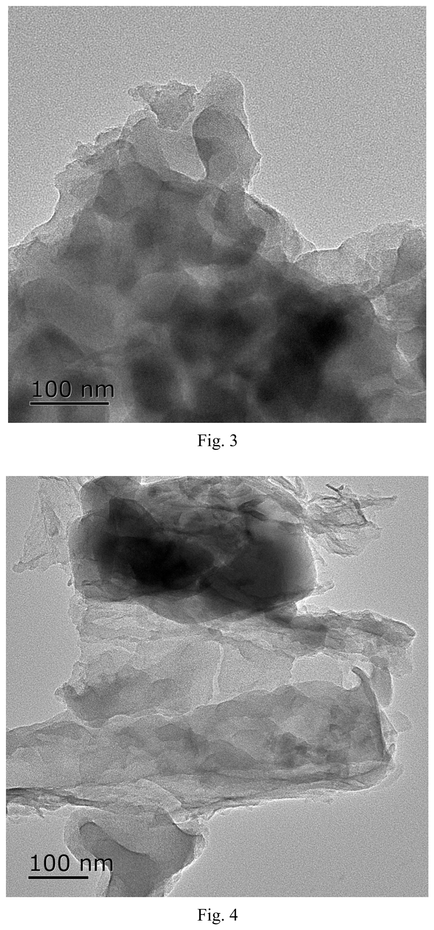

This example provided a method for preparing dimethyl carbonate:
Preparation of catalyst for preparing dimethyl carbonate: In a 500 ml three-necked flask, 50 g of chloromethylated styrene resin and 200 ml (189.6 g) of N,N-dimethylformamide were added, then stirred at 500 rpm for 2 h, the temperature was raised to 90°C, 50 g of imidazole was added, and the temperature was maintained for reaction for 12 h. After cooling, the product was separated, washed with deionized water, then the product was dried at 100°C for 24 h to obtain a modified resin. Then, in another 500 ml three-necked flask, 20 g of the modified resin, and 100 g of a solution prepared by adding 8.8 g of magnesium nitrate to water were added. The mixture was stirred at 500 rpm at 80°C for 6 h, placed in an oven and dried at 100°C for 14 h, then transferred to a muffle furnace, and calcined at 500°C for 5 h under a nitrogen atmosphere to obtain catalyst C1. The mass percentage of magnesium oxide in catalyst C1 was 10%. The SEM image of catalyst C1 is shown in Figure 1, and the TEM image of catalyst C1 is shown in Figure 3. The nitrogen content of the catalyst was 2.6%, the pore size was 150 nm, and the proportion of magnesium oxide within the pore channels was 86.7%.

During the above catalyst preparation, the chloromethylated styrene resin was spherical with a particle size of 0.5 mm.

Preparation of dimethyl carbonate: 22.0 g (0.25 mol) of ethylene carbonate, 64.0 g (2 mol) of methanol, and 4.4 g of catalyst C1 were placed into a 100 ml autoclave, and reacted at 120°C for 4 h. The molar ratio of methanol to ethylene carbonate was 8:1, and the mass ratio of catalyst C1 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 80.4%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

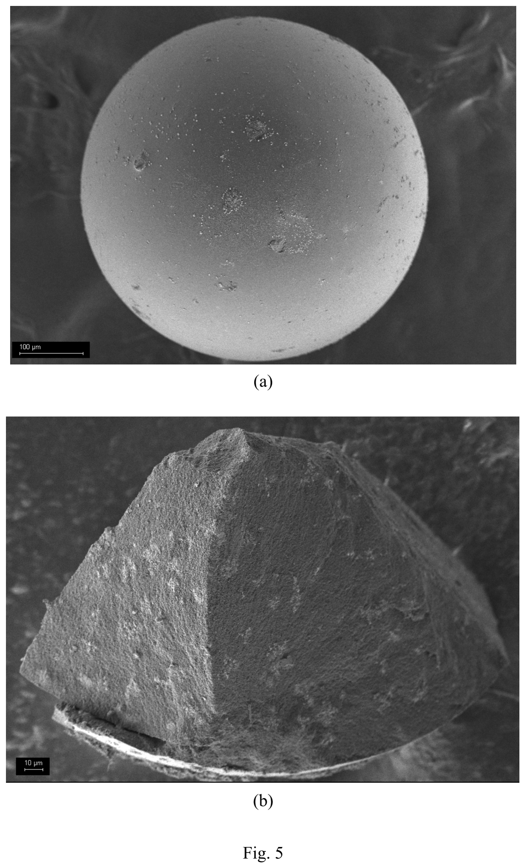

In Figure 5, (a) showed a scanning electron micrograph of catalyst C1 prepared in Example 1 of the present invention at a 100-micron scale; (b) showed a scanning electron micrograph of the interior of the catalyst prepared in Example 1 of the present invention at a 10-micron scale. It could be seen that the outer surface of the carbon support was smooth, and magnesium oxide was mainly distributed on the internal surface of the carbon support.

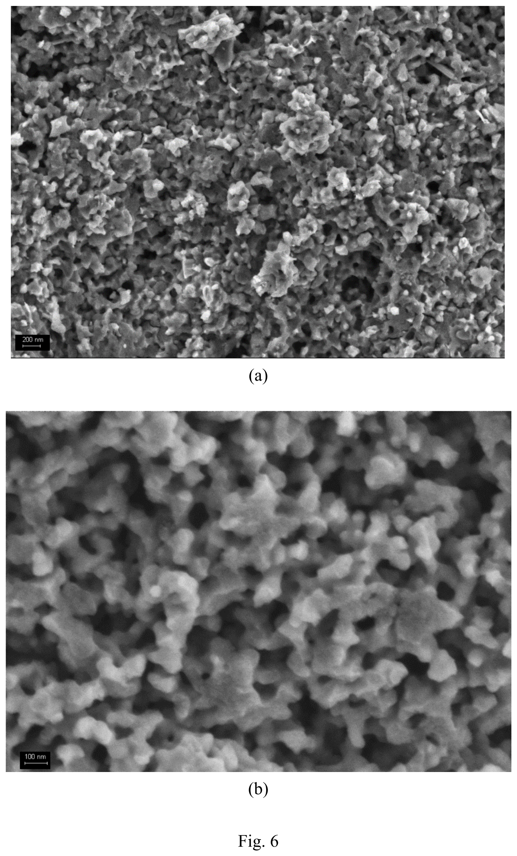

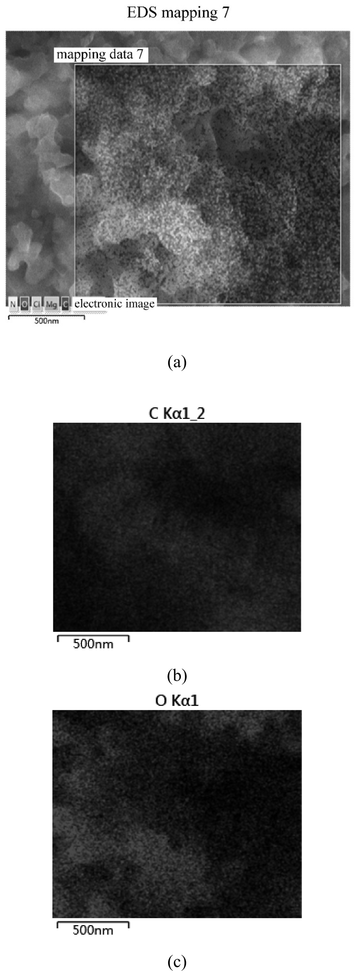

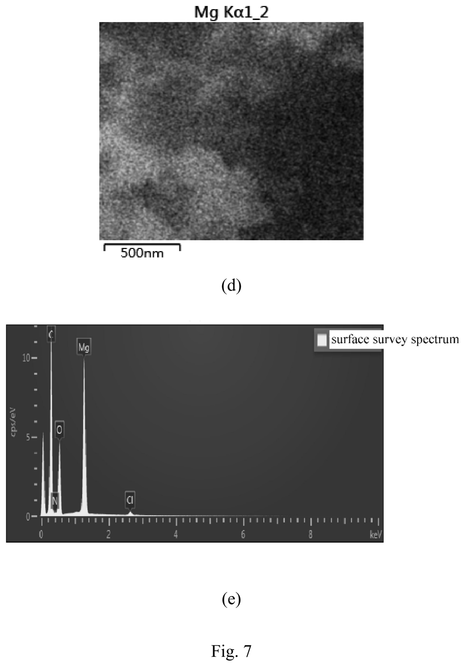

In Figure 6, (a) showed a scanning electron micrograph of the interior of catalyst C1 prepared in Example 1 of the present invention at a 200-nanometer scale; (b) showed a scanning electron micrograph of the interior of catalyst C1 prepared in Example 1 of the present invention at a 100-nanometer scale. It could be seen that the pore channels were filled to a certain extent, indicating the internal deposition of magnesium oxide. Figure 7 showed an Energy Dispersive Spectroscopy mapping and EDX spectrum of catalyst C1 prepared in Example 1 of the present invention; (a) showed a selected area mapping; (b) showed a carbon element distribution map; (c) showed an oxygen element distribution map; (d) showed a magnesium element distribution map; (e) showed an EDX energy spectrum. It could be seen that magnesium oxide was mainly distributed in the interior of the carbon support, and the EDX relative abundance was high.

Example 2

This example provided a method for preparing dimethyl carbonate:
Preparation of catalyst for preparing dimethyl carbonate: In a 500 ml three-necked flask, 50 g of chloromethylated styrene resin and 200 ml (189.6 g) of N,N-dimethylformamide were added, then stirred at 500 rpm for 2 h, the temperature was raised to 90°C, 50 g of imidazole was added, and the temperature was maintained for reaction for 12 h. After cooling, the product was separated, washed with deionized water, then the product was dried at 100°C for 24 h to obtain a modified resin. Then, in another 500 ml three-necked flask, 20 g of the modified resin, and 100 g of a solution prepared by adding 17.6 g of magnesium nitrate to water were added. The mixture was stirred at 600 rpm at 50°C for 8 h, placed in an oven and dried at 100°C for 24 h, then transferred to a muffle furnace, and calcined at 400°C for 8 h under a nitrogen atmosphere to obtain catalyst C2. The mass percentage of magnesium oxide in catalyst C2 was 20%. The nitrogen content of the catalyst was 2.2%, the pore size was 200 nm, and the proportion of magnesium oxide within the pore channels was 71.1%.

During the above catalyst preparation, the chloromethylated styrene resin was spherical with a particle size of 0.9 mm.

Preparation of dimethyl carbonate: 22.0 g (0.25 mol) of ethylene carbonate, 48.0 g (1.5 mol) of methanol, and 4.4 g of catalyst C2 were placed into a 100 ml autoclave, and reacted at 110°C for 6 h. The molar ratio of methanol to ethylene carbonate was 6:1, and the mass ratio of catalyst C2 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 74.5%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 74%, and the dimethyl carbonate selectivity remained at 99.9%.

Example 3

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 80°C, 40 g of imidazole was added, and the temperature was maintained for reaction for 12 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 26.4 g of magnesium nitrate to water were added. The mixture was stirred at 300 rpm at 50°C for 8 h, placed in an oven and dried at 100°C for 24 h, then transferred to a muffle furnace, and calcined at 500°C for 4 h under a nitrogen atmosphere to obtain catalyst C3. The content of magnesium oxide in catalyst C3 was 30%. The nitrogen content of the catalyst was 1.7%, the pore size was 250 nm, and the proportion of magnesium oxide within the pore channels was 63.3%.

During the above catalyst preparation, the chloromethylated styrene resin was spherical with a particle size of 0.7 mm.

Preparation of dimethyl carbonate: 22.0 g (0.25 mol) of ethylene carbonate, 32.0 g (1.0 mol) of methanol, and 4.4 g of catalyst C3 were placed into a 100 ml autoclave, and reacted at 110°C for 10 h. The molar ratio of methanol to ethylene carbonate was 4:1, and the mass ratio of catalyst C3 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 61.4%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 60%, and the dimethyl carbonate selectivity remained at 99.9%.

Example 4

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of imidazole was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 35.2 g of magnesium nitrate to water were added. The mixture was stirred at 400 rpm at 50°C for 2 h, placed in an oven and dried at 100°C for 24 h, then transferred to a muffle furnace, and calcined at 500°C for 4 h under a nitrogen atmosphere to obtain catalyst C4. The content of magnesium oxide in catalyst C4 was 40%. The nitrogen content of the catalyst was 1.8%, the pore size was 300 nm, and the proportion of magnesium oxide within the pore channels was 63.3%.

During the above catalyst preparation, the chloromethylated styrene resin was spherical with a particle size of 1.0 mm.

Preparation of dimethyl carbonate: 22.0 g (0.25 mol) of ethylene carbonate, 64.0 g (2 mol) of methanol, and 8.8 g of catalyst C4 were placed into a 100 ml autoclave, and reacted at 90°C for 8 h. The molar ratio of methanol to ethylene carbonate was 8:1, and the mass ratio of catalyst C4 to ethylene carbonate was 0.4:1. After the reaction, the autoclave was cooled to room temperature and vented; the liquid phase product was taken for gas chromatographic analysis and calculation. The result was as follows: the ethylene carbonate conversion rate was 80.9%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 5

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of imidazole was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 26.4 g of magnesium nitrate to water were added. The mixture was stirred at 400 rpm at 50°C for 2 h, placed in an oven and dried at 100°C for 24 h, then transferred to a muffle furnace, and calcined at 500°C for 4 h under a nitrogen atmosphere to obtain catalyst C5. The content of magnesium oxide in catalyst C5 was 30%. The nitrogen content of the catalyst was 1.8%, the pore size was 180 nm, and the proportion of magnesium oxide within the pore channels was 64.4%.

During the above catalyst preparation, the chloromethylated styrene resin was spherical with a particle size of 1.0 mm.

Preparation of dimethyl carbonate: 22.0 g (0.25 mol) of ethylene carbonate, 64.0 g (2 mol) of methanol, and 6.6 g of catalyst C5 were placed into a 100 ml autoclave, and reacted at 80°C for 10 h. The molar ratio of methanol to ethylene carbonate was 8:1, and the mass ratio of catalyst C5 to ethylene carbonate was 0.3:1. After the reaction, the autoclave was cooled to room temperature and vented; the liquid phase product was taken for gas chromatographic analysis and calculation. The result was as follows: the ethylene carbonate conversion rate was 80.5%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 6

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of thiophene was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 26.4 g of magnesium nitrate to water were added. The mixture was stirred at 400 rpm at 50°C for 2 h, placed in an oven and dried at 100°C for 24 h, then transferred to a muffle furnace, and calcined at 500°C for 4 h under a nitrogen atmosphere to obtain catalyst C6. The content of magnesium oxide in catalyst C6 was 30%. The sulfur content of the catalyst was 2.7%, the pore size was 100 nm, and the proportion of magnesium oxide within the pore channels was 66.4%.

During the above catalyst preparation, the chloromethylated styrene resin was spherical with a particle size of 1 mm.

Preparation of dimethyl carbonate: 22.0 g (0.25 mol) of ethylene carbonate, 64.0 g (2 mol) of methanol, and 8.8 g of catalyst C6 were placed into a 100 ml autoclave, and reacted at 70°C for 8 h. The molar ratio of methanol to ethylene carbonate was 8:1, and the mass ratio of catalyst C6 to ethylene carbonate was 0.4:1. After the reaction, the autoclave was cooled to room temperature and vented; the liquid phase product was taken for gas chromatographic analysis and calculation. The result was as follows: the ethylene carbonate conversion rate was 80.2%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 7

This example provided a method for preparing dimethyl carbonate:
Preparation of catalyst for preparing dimethyl carbonate: Then, in another 500 ml three-necked flask, 20 g of the modified resin, and 100 g of a solution prepared by adding 8 g of calcium nitrate to water were added. The mixture was stirred at 300 rpm at 50°C for 2 h, placed in an oven and dried at 100°C for 24 h, then transferred to a muffle furnace, and calcined at 500°C for 4 h under a nitrogen atmosphere to obtain catalyst C7. The content of calcium oxide in catalyst C7 was 20%. The nitrogen content of the catalyst was 2.2%, the pore size was 120 nm, and the proportion of calcium oxide within the pore channels was 68.8%.

During the above catalyst preparation, the chloromethylated styrene resin was spherical with a particle size of 1.0 mm.

Preparation of dimethyl carbonate: 22.0 g (0.25 mol) of ethylene carbonate, 64.0 g (2 mol) of methanol, and 8.8 g of catalyst C7 were placed into a 100 ml autoclave, and reacted at 70°C for 8 h. The molar ratio of methanol to ethylene carbonate was 8:1, and the mass ratio of catalyst C7 to ethylene carbonate was 0.4:1. After the reaction, the autoclave was cooled to room temperature and vented; the liquid phase product was taken for gas chromatographic analysis and calculation. The result was as follows: the ethylene carbonate conversion rate was 80.4%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 8

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of triphenylphosphine was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 80 g of a solution prepared by adding 17.6 g of magnesium nitrate to water were added. The mixture was stirred at 500 rpm at 50°C for 2 h, placed in an oven and dried at 100°C for 24 h, then transferred to a muffle furnace, and calcined at 500°C for 4 h under a nitrogen atmosphere to obtain catalyst C8. The content of magnesium oxide in catalyst C8 was 20%. The phosphorus content of the catalyst was 2.2%, the pore size was 150 nm, and the proportion of magnesium oxide within the pore channels was 71.6%.

During the above catalyst preparation, the chloromethylated styrene resin was spherical with a particle size of 1.0 mm.

Preparation of dimethyl carbonate: 22.0 g (0.25 mol) of ethylene carbonate, 64.0 g (2 mol) of methanol, and 8.8 g of catalyst C8 were placed into a 100 ml autoclave, and reacted at 90°C for 8 h. The molar ratio of methanol to ethylene carbonate was 8:1, and the mass ratio of catalyst C8 to ethylene carbonate was 0.4:1. After the reaction, the autoclave was cooled to room temperature and vented; the liquid phase product was taken for gas chromatographic analysis and calculation. The result was as follows: the ethylene carbonate conversion rate was 80.3%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 9

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of piperazine was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 11.4 g of aluminum nitrate to water were added. The mixture was stirred at 500 rpm at 50°C for 2 h, placed in an oven and dried at 100°C for 24 h, then transferred to a muffle furnace, and calcined at 600°C for 4 h under a nitrogen atmosphere to obtain catalyst C9. The content of aluminum oxide in catalyst C9 was 20%. The nitrogen content of the catalyst was 0.8%, the pore size was 100 nm, and the proportion of aluminum oxide within the pore channels was 67.6%.

During the above catalyst preparation, the chloromethylated styrene resin was spherical with a particle size of 1.2 mm.

Preparation of dimethyl carbonate: 22.0 g (0.25 mol) of ethylene carbonate, 64.0 g (2 mol) of methanol, and 8.8 g of catalyst C9 were placed into a 100 ml autoclave, and reacted at 90°C for 8 h. The molar ratio of methanol to ethylene carbonate was 8:1, and the mass ratio of catalyst C9 to ethylene carbonate was 0.4:1. After the reaction, the autoclave was cooled to room temperature and vented; the liquid phase product was taken for gas chromatographic analysis and calculation. The result was as follows: the ethylene carbonate conversion rate was 80.6%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 10

This example provided a method for preparing dimethyl carbonate:
Preparation of catalyst for preparing dimethyl carbonate: In a 500 ml three-necked flask, 50 g of chloromethylated styrene resin and 300 ml (284.4 g) of N,N-dimethylformamide were added, then stirred at 800 rpm for 2 h, the temperature was raised to 70°C, 30 g of 1-methylimidazole was added, and the temperature was maintained for reaction for 15 h. After cooling, the product was separated, washed with deionized water, then the product was dried at 120°C for 24 h to obtain a modified resin. Then, in another 500 ml three-necked flask, 20 g of the modified resin, and 100 g of a solution prepared by adding 26.4 g of magnesium nitrate to water were added. The mixture was stirred at 500 rpm at 50°C for 2 h, placed in an oven and dried at 100°C for 24 h, then transferred to a muffle furnace, and calcined at 500°C for 4 h under a nitrogen atmosphere to obtain catalyst C10. The content of magnesium oxide in catalyst C10 was 30%. The nitrogen content of the catalyst was 1.6%, the pore size was 150 nm, and the proportion of magnesium oxide within the pore channels was 66.5%.

During the above catalyst preparation, the chloromethylated styrene resin was spherical with a particle size of 1.0 mm.

Preparation of dimethyl carbonate: 22.0 g (0.25 mol) of ethylene carbonate, 64.0 g (2 mol) of methanol, and 6.6 g of catalyst C10 were placed into a 100 ml autoclave, and reacted at 80°C for 10 h. The molar ratio of methanol to ethylene carbonate was 8:1, and the mass ratio of catalyst C10 to ethylene carbonate was 0.3:1. After the reaction, the autoclave was cooled to room temperature and vented; the liquid phase product was taken for gas chromatographic analysis and calculation. The result was as follows: the ethylene carbonate conversion rate was 80.5%, and the dimethyl carbonate selectivity was 99.9%.

Further, after the reaction, the granular catalyst was filtered and recovered from the reaction mixture, washed with deionized water, placed in an oven and dried at 100°C for 24 h, then reused. The conditions were consistent with the above dimethyl carbonate preparation process. After reaction, the liquid phase product was taken for gas chromatographic analysis and the ethylene carbonate conversion rate was calculated. After repeated recovery and reuse of the catalyst 10 times, the ethylene carbonate conversion rate was all greater than 80%, and the dimethyl carbonate selectivity remained at 99.9%.

Example 11

This example provided a method for preparing ethyl methyl carbonate:
Preparation of ethyl methyl carbonate: 22.0 g (0.25 mol) of dimethyl carbonate, 46.0 g (1 mol) of ethanol, and 6.75 g of catalyst C10 were placed into a 100 ml autoclave, and reacted at 100°C for 10 h. The molar ratio of ethanol to dimethyl carbonate was 4:1, and the mass ratio of catalyst C10 to dimethyl carbonate was 0.3:1. After the reaction, the autoclave was cooled to room temperature and vented; the liquid phase product was taken for gas chromatographic analysis and calculation. The result was as follows: the dimethyl carbonate conversion rate was 91.6%, and the ethyl methyl carbonate selectivity was 92.4%.

Example 12

This example provided a method for preparing ethyl methyl carbonate:
Preparation of ethyl methyl carbonate: 22.0 g (0.25 mol) of dimethyl carbonate, 11.5 g (0.25 mol) of ethanol, and 4.5 g of catalyst C10 were placed into a 100 ml autoclave, and reacted at 100°C for 10 h. The molar ratio of ethanol to dimethyl carbonate was 1:1, and the mass ratio of catalyst C10 to dimethyl carbonate was 0.2:1. After the reaction, the autoclave was cooled to room temperature and vented; the liquid phase product was taken for gas chromatographic analysis and calculation. The result was as follows: the dimethyl carbonate conversion rate was 64.5%, and the ethyl methyl carbonate selectivity was 73.1%.

Comparative Example 1

This comparative example provided a method for preparing dimethyl carbonate:
Preparation of catalyst for preparing dimethyl carbonate: In a 500 ml three-necked flask, 20 g of chloromethylated styrene resin, and 100 g of a solution prepared by adding 8.8 g of magnesium nitrate to water were added. The mixture was stirred at 80°C for 6 h, placed in an oven and dried at 100°C for 14 h, then transferred to a muffle furnace, and calcined at 500°C for 5 h under a nitrogen atmosphere to obtain catalyst S1. The mass percentage of magnesium oxide in catalyst S1 was 10%. The SEM image of catalyst S1 is shown in Figure 2, and the TEM image of catalyst S1 is shown in Figure 4. It was found by measurement and calculation that there was almost no active metal component within the pore channels.

During the above catalyst preparation, the chloromethylated styrene resin was spherical with a particle size of 0.5 mm.

Preparation of dimethyl carbonate: 22.0 g (0.25 mol) of ethylene carbonate, 64.0 g (2 mol) of methanol, and 4.4 g of catalyst S1 were placed into a 100 ml autoclave, and reacted at 120°C for 4 h. The molar ratio of methanol to ethylene carbonate was 8:1, and the mass ratio of catalyst S1 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 59.6%, and the dimethyl carbonate selectivity was 99.8%.

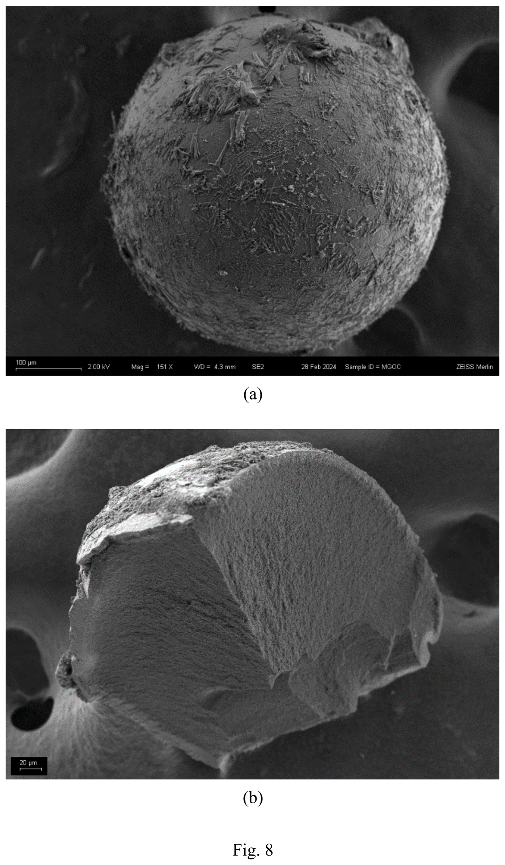

In Figure 8, (a) showed a scanning electron micrograph of catalyst S1 prepared in Comparative Example 1 of the present invention at a 100-micron scale; (b) showed a scanning electron micrograph of the interior of the catalyst S1 prepared in Comparative Example 1 of the present invention at a 20-micron scale. It could be seen that the outer surface of the carbon support was rough, with magnesium oxide attached thereon, while almost no magnesium oxide was seen on the internal surface of the carbon support.

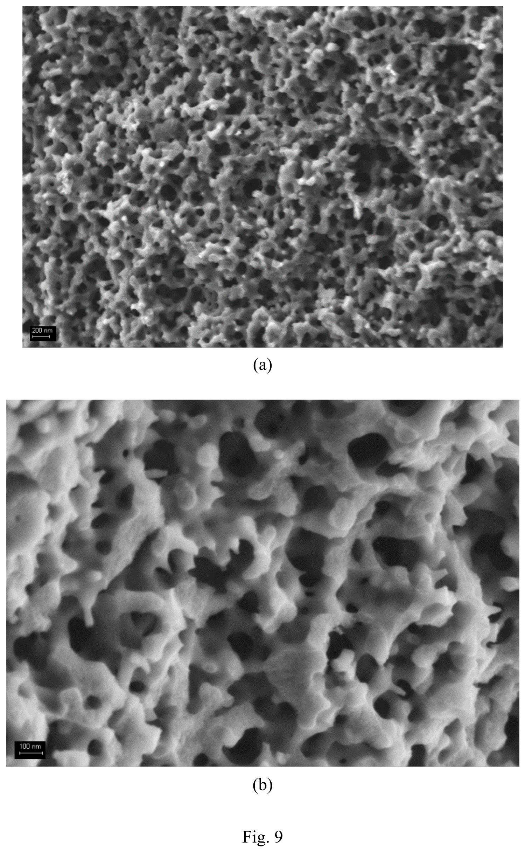

In Figure 9, (a) showed a scanning electron micrograph of catalyst S1 prepared in Comparative Example 1 of the present invention at a 200-nanometer scale; (b) showed a scanning electron micrograph of the interior of the catalyst S1 prepared in Comparative Example 1 of the present invention at a 100-nanometer scale. It could be seen that the interior of the carbon support maintained its pore channel structure, and there was no entry of magnesium oxide.

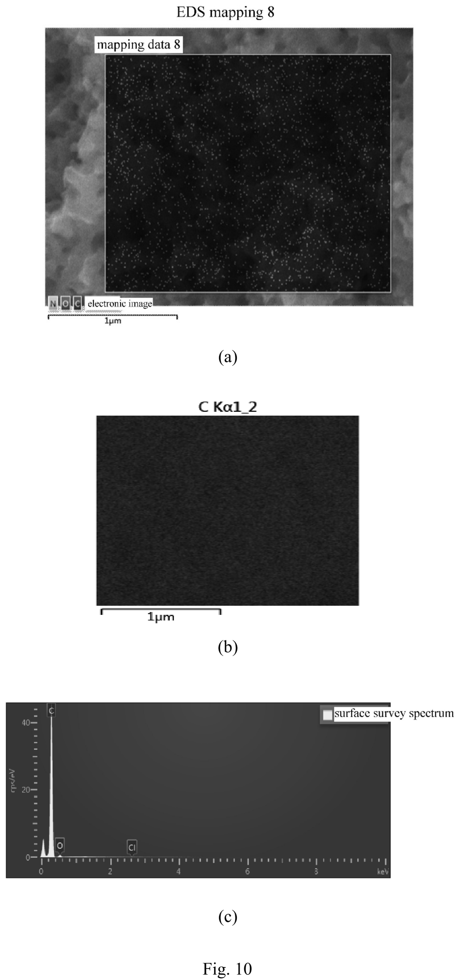

Figure 10 showed an energy-dispersive X-ray spectroscopy area scan and EDX spectrum of catalyst C1 prepared in Comparative Example 1 of the present invention; (a) showed a selected area surface scan spectrum; (b) showed a carbon element distribution map; (c) showed an EDX energy spectrum. It could be seen that there was basically no distribution of magnesium oxide within the pore channels of the carbon support, and the EDX result showed substantially no detectable magnesium.

Comparative Example 2

This comparative example provided a method for preparing dimethyl carbonate:
Preparation of catalyst for preparing dimethyl carbonate: 20 g of magnesium nitrate was transferred to a muffle furnace and calcined at 500°C for 5 h under a nitrogen atmosphere to obtain a powdery catalyst S2.

Preparation of dimethyl carbonate: 22.0 g (0.25 mol) of ethylene carbonate, 64.0 g (2 mol) of methanol, and 4.4 g of catalyst S2 were placed into a 100 ml autoclave, and reacted at 120°C for 4 h. The molar ratio of methanol to ethylene carbonate was 8:1, and the mass ratio of catalyst S2 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 32.7%, and the dimethyl carbonate selectivity was 99.9%.

Comparative Example 3

This comparative example provided a method for preparing dimethyl carbonate:
Preparation of catalyst for preparing dimethyl carbonate: In a 500 ml three-necked flask, 20 g of styrene resin, and 100 g of a solution prepared by adding 8.8 g of magnesium nitrate to water were added. The mixture was stirred at 80°C for 6 h, placed in an oven and dried at 100°C for 14 h, then transferred to a muffle furnace, and calcined at 500°C for 5 h under a nitrogen atmosphere to obtain catalyst S3. The mass percentage of magnesium oxide in catalyst S3 was 6.5%. It was found by measurement and calculation that there was almost no active metal component within the pore channels. During the above catalyst preparation, the chloromethylated styrene resin was spherical with a particle size of 0.5 mm.

Preparation of dimethyl carbonate: 22.0 g (0.25 mol) of ethylene carbonate, 64.0 g (2 mol) of methanol, and 4.4 g of catalyst S3 were placed into a 100 ml autoclave, and reacted at 120°C for 4 h. The molar ratio of methanol to ethylene carbonate was 8:1, and the mass ratio of catalyst S3 to ethylene carbonate was 0.2:1. After the reaction, the autoclave was cooled to room temperature and vented; the liquid phase product was taken for gas chromatographic analysis and calculation. The result was as follows: the ethylene carbonate conversion rate was 39.4%, and the dimethyl carbonate selectivity was 99.9%.

It should be noted that the examples described above are only for explaining the present invention and do not constitute any limitation on the present invention. The present invention has been described by reference to typical examples, but it should be understood that the words and expressions used are descriptive and explanatory, not restrictive. The present invention may be modified within the scope of the claims as required, and may be revised without departing from the scope and spirit of the present invention. Although the present invention described herein involves specific methods, materials, and examples, it does not mean that the present invention is limited to the specific examples disclosed therein. On the contrary, the present invention may extend to all other methods and applications having the same function.

**Claims**

1. A porous carbon support-loaded catalyst, **characterized in that** it comprises a porous carbon support, an active metal component, and at least one heteroatom selected from nitrogen, sulfur, and phosphorus, wherein, relative to the total mass of the catalyst, the content of the porous carbon support is 50% to 99%, the content of the active metal component calculated as active metal oxide is 0.1% to 50%, and the content of the heteroatom(s) calculated as element is 0.1% to 5%; said active metal is at least one selected from magnesium, aluminum, and calcium; and calculated as active metal oxide, the content of the active metal component distributed within porous channels of the porous carbon support accounts for more than 50% relative to the total content of all active metal components in the catalyst.

2. The catalyst according to claim 1, **characterized in that** the particle size of said porous carbon support is 0.1 mm to 3 mm, preferably 0.3 mm to 2 mm; and the pore size of the porous carbon support is 5 to 500 nm, preferably 50 to 300 nm.

3. The catalyst according to claim 1 or 2, **characterized in that** it satisfies at least one of the following conditions:

> calculated as active metal oxide, the content of the active metal component distributed within porous channels of the porous carbon support accounts for 55% or more, preferably 60% or more, further preferably 65% or more, most preferably 70% or more, relative to the total content of all active metal components in the catalyst;
> relative to the total mass of the catalyst, the content of the porous carbon support is 55% to 90%, preferably 60% to 85%, and the content of the active metal component calculated as active metal oxide is 5% to 45%, preferably 10% to 40%; and
> the content of the heteroatom(s) calculated as element is 0.3% to 4.5%, preferably 0.5% to 4%.

4. The catalyst according to any one of claims 1 to 3, **characterized in that**, calculated as active metal oxide, the content of the active metal component distributed on the outer surface of the porous carbon support as determined by XPS accounts for less than 50%, preferably 45% or less, more preferably 40% or less, further preferably 35% or less, most preferably 30% or less, relative to the total content of all active metal components in the catalyst.

5. A process for preparing a porous carbon support-loaded catalyst, comprising the following steps:

> (1) a modification step, in which a haloalkylated resin is contacted with a modifier to perform a modification, thereby obtaining a modified haloalkylated resin;
> (2) a contact step, in which the modified haloalkylated resin is contacted with a metal precursor of an active metal, thereby obtaining a contact product; and
> (3) a calcining step, in which the contact product is optionally dried and then calcined in an inactive gas atmosphere,

said modifier is at least one compound selected from nitrogen-containing compounds, sulfur-containing compounds, and phosphorus-containing compounds, and said active metal is at least one selected from magnesium, aluminum, and calcium.

6. The process according to claim 5, wherein step (1) satisfies at least one of the following conditions:

> the haloalkylated resin is a halomethylated resin, preferably a chloromethylated resin, more preferably a chloromethylated styrene resin;
> the modifier is nitrogen-containing organic compounds, sulfur-containing organic compounds or phosphorus-containing organic compounds, preferably alkylamines (for example methylamine, ethylamine, hexylamine, propylamine, dimethylamine, diethylamine), 5- or 6-membered nitrogen-containing heterocyclic aromatic compounds, thiophene compounds or triphenylphosphine, more preferably 5- or 6-membered nitrogen-containing heterocyclic aromatic compounds optionally substituted by $C_1$-$C_6$alkyl, thiophene compunds optionally substituted by $C_1$-$C_6$alkyl or triphenylphosphine, particularly preferably at least one of piperazine optionally substituted by $C_1$-$C_6$alkyl, pyridine optionally substituted by $C_1$-$C_6$alkyl, imidazole optionally substituted by $C_1$-$C_6$alkyl, quinoline optionally substituted by $C_1$-$C_6$alkyl, pyrrole optionally substituted by $C_1$-$C_6$alkyl, indole optionally substituted by $C_1$-$C_6$alkyl, carbazole optionally substituted by $C_1$-$C_6$alkyl, isoindole optionally substituted by $C_1$-$C_6$alkyl, thiazole optionally substituted by $C_1$-$C_6$alkyl, thiophene optionally substituted by $C_1$-$C_6$alkyl, triphenylphosphine;
> the mass ratio of said haloalkylated resin to the modifier is 1:(0.1-1.2), preferably 1:(0.15-1);
> the modification step is carried out in a solvent, wherein said solvent is at least one selected from toluene, methanol, ethanol, n-propanol, n-butanol, isobutanol, tert-butanol, tetrahydrofuran, dichloromethane, trichloromethane, N,N-dimethylformamide, ether, and water, and the mass ratio of the haloalkylated resin to the solvent is 1:(1-10), preferably 1:(2-8);
> the temperature of the modification step is 30°C to 150°C, preferably 40°C to 120°C;
> the modification time of the modification step is 1 h to 24 h, preferably 2 h to 18 h;
> the modification step is performed under mixing and stirring, wherein the mixing and stirring rate is 50 rpm to 1000 rpm, preferably 100 rpm to 800 rpm;
> in the modification step, the obtained modified haloalkylated resin is washed and dried, wherein the drying conditions include: the drying temperature is 80°C to 180°C, the drying time is 1 h to 48 h.

7. The process according to claim 5 or 6, wherein step (2) satisfies at least one of the following conditions:

the metal precursor of the active metal is in at least one form of nitrate, chloride, and sulfate of the active metal;
the mass ratio of the modified haloalkylated resin to the metal precursor of the active metal is 1:0.1 to 3, preferably 1:0.2 to 2;
the metal precursor of the active metal is kept in form of solution to contact the modified haloalkylated resin obtained in step (1), wherein the solvent is at least one selected from toluene, methanol, ethanol, n-propanol, n-butanol, isobutanol, tert-butanol, tetrahydrofuran, dichloromethane, trichloromethane, N,N-dimethylformamide, ether, and water;
step (2) is performed under mixing and stirring conditions, wherein the mixing and stirring rate is 100 rpm to 800 rpm, preferably 200 rpm to 600 rpm; the mixing and stirring temperature is 30°C to 100°C, preferably 50°C to 80°C; and the mixing and stirring time is 1 h to 10 h, preferably 2 h to 8 h.

8. The process according to any one of claims 5-7, wherein step (3) satisfies at least one of the following conditions:

the contact product obtained in step (2) is dried, wherein the drying conditions include: the temperature is 80°C to 180°C, preferably 90°C to 150°C; the time is 1 h to 48 h, preferably 2 h to 36 h;
the inactive gas is at least one of nitrogen gas and noble gas;
the calcining temperature is 200°C to 1000°C, preferably 250°C to 900°C; and the calcining time is 1 h to 48 h, preferably 2 h to 24 h.

9. A method for preparing a dialkyl carbonate represented by the following formula (I), **characterized in that**, in the presence of the catalyst according to any one of claims 1 to 4 or a catalyst prepared by the process according to any one of claims 5 to 8, a carbonate represented by the following formula (II) and a lower alcohol represented by the following formula (III) are reacted to obtain the dialkyl carbonate represented by formula (I);

$$R_1\text{-OCOO-}R_1 \qquad (I)$$

in formula (I), each $R_1$ is independently selected from $C_1$-$C_6$ alkyl;

$$R_2\text{-OCOO-}R_2 \qquad (II)$$

in formula (II), each $R_2$ is independently selected from $C_1$-$C_6$ alkyl, and at least one $R_2$ is different from at least one $R_1$; or the two $R_2$ are bonded to each other to form a $C_2$-$C_6$ alkylene group;

$$R_1\text{-OH} \qquad (III)$$

in formula (III), $R_1$ is selected from $C_1$-$C_6$ alkyl.

10. The method according to claim 9, **characterized in that** it satisfies at least one of the following conditions:

the reaction temperature is 40°C to 130°C, preferably 50°C to 120°C;
the reaction time is 1 h to 15 h, preferably 2 h to 12 h.

11. The method according to claim 9 or 10, **characterized in that** it satisfies at least one of the following conditions:

the molar ratio of the lower alcohol represented by formula (III) to the carbonate represented by formula (II) is (0.5-15):1, preferably (1-12):1;
the mass ratio of the catalyst to the carbonate represented by formula (II) is (0.001-0.5):1, preferably (0.05-0.5):1;
in formula (I), the two $R_1$ are identical to each other; and
in formula (II), the two $R_2$ are bonded to each other to form a $C_2$-$C_6$ alkylene group.

12. The method according to any one of claims 9-11, **characterized in that** it satisfies at least one of the following conditions:

the carbonate represented by formula (II) is at least one of ethylene carbonate and propylene carbonate, the lower alcohol is at least one of methanol, ethanol, propanol, isopropanol, and butanol;
the dialkyl carbonate represented by formula (I) is at least one of dimethyl carbonate, diethyl carbonate, ethyl methyl carbonate, dipropyl carbonate, and dibutyl carbonate.

Fig. 1

Fig. 2

Fig. 3

Fig. 4

(a)

(b)

Fig. 5

(a)

(b)

Fig. 6

EDS mapping 7

(a)

C Kα1_2

(b)

O Kα1

(c)

Mg Kα1_2

500nm

(d)

(e)

Fig. 7

(a)

(b)

Fig. 8

(a)

(b)

Fig. 9

EDS mapping 8

(a)

C Kα1_2

(b)

(c)

Fig. 10

## INTERNATIONAL SEARCH REPORT

| International application No. |
|---|
| **PCT/CN2024/101940** |

| A. | CLASSIFICATION OF SUBJECT MATTER |
|---|---|

B01J21/18(2006.01)i; B01J35/60(2024.01)i; B01J23/02(2006.01)i; B01J21/04(2006.01)i; C07C27/00(2006.01)i; C07C29/128(2006.01)i; C07C31/20(2006.01)i; C07C68/065(2020.01)i; C07C69/96(2006.01)i

According to International Patent Classification (IPC) or to both national classification and IPC

| B. | FIELDS SEARCHED |
|---|---|

Minimum documentation searched (classification system followed by classification symbols)

IPC: B01J21/-; B01J35/-; B01J23/-; C07C27/-; C07C29/-; C07C31/-; C07C68/-; C07C69/-;

Documentation searched other than minimum documentation to the extent that such documents are included in the fields searched

Electronic data base consulted during the international search (name of data base and, where practicable, search terms used)

CNTXT, CNKI, ENTXT, VEN, Web of Science: 镁, 钙, 铝, 多孔碳, 树脂, 氯甲基苯乙烯, 改性, 氮, 硫, 磷, 孔道, 酯交换, 碳酸二甲酯, 碳酸烷基酯, magnesium, Mg, calcium, Ca, aluminum, Al, porous carbon, resins, chloromethylstyrene, modified, nitrogen, sulfur, phosphorus, pore, tunnel, transesterification, dimethyl carbonate, alkyl carbonate

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 107915574 A (CHINA PETROCHEMICAL CORP. et al.) 17 April 2018 (2018-04-17) description, paragraphs 5-10 | 1-12 |
| A | CN 112169823 A (CHANGCHUN INSTITUTE OF APPLIED CHEMISTRY, CHINESE ACADEMY OF SCIENCES) 05 January 2021 (2021-01-05) entire document | 1-12 |
| A | CN 115888813 A (CHINA PETROCHEMICAL CORP.) 04 April 2023 (2023-04-04) entire document | 1-12 |
| A | JP 2004010571 A (MITSUBISHI CHEMICAL CORP.) 15 January 2004 (2004-01-15) entire document | 1-12 |
| A | US 2009203933 A1 (CATALYTIC DISTILLATION TECHNOLOGIES) 13 August 2009 (2009-08-13) entire document | 1-12 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 September 2024** | **01 October 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/101940**

| Patent document cited in search report | | | Publication date (day/month/year) | Patent family member(s) | | | Publication date (day/month/year) |
|---|---|---|---|---|---|---|---|
| CN | 107915574 | A | 17 April 2018 | None | | | |
| CN | 112169823 | A | 05 January 2021 | None | | | |
| CN | 115888813 | A | 04 April 2023 | None | | | |
| JP | 2004010571 | A | 15 January 2004 | None | | | |
| US | 2009203933 | A1 | 13 August 2009 | TW | 201329039 | A | 16 July 2013 |
| | | | | TWI | 491589 | B | 11 July 2015 |
| | | | | RU | 2447056 | C1 | 10 April 2012 |
| | | | | WO | 2009102556 | A2 | 20 August 2009 |
| | | | | WO | 2009102556 | A3 | 23 December 2009 |
| | | | | WO | 2009102556 | A8 | 02 September 2010 |
| | | | | JP | 2013237046 | A | 28 November 2013 |
| | | | | JP | 5718985 | B2 | 13 May 2015 |
| | | | | KR | 20120030605 | A | 28 March 2012 |
| | | | | KR | 101409655 | B1 | 18 June 2014 |
| | | | | TW | 200946497 | A | 16 November 2009 |
| | | | | TWI | 412517 | B | 21 October 2013 |
| | | | | EP | 2257520 | A2 | 08 December 2010 |
| | | | | EP | 2257520 | A4 | 04 December 2013 |
| | | | | EP | 2257520 | B1 | 22 August 2018 |
| | | | | US | 2011045965 | A1 | 24 February 2011 |
| | | | | US | 8361919 | B2 | 29 January 2013 |
| | | | | ES | 2705007 | T3 | 21 March 2019 |
| | | | | BRPI | 0908389 | A2 | 11 August 2015 |
| | | | | BRPI | 0908389 | B1 | 12 February 2019 |
| | | | | JP | 2014098005 | A | 29 May 2014 |
| | | | | JP | 5926300 | B2 | 25 May 2016 |
| | | | | US | 2011046409 | A1 | 24 February 2011 |
| | | | | US | 8222444 | B2 | 17 July 2012 |
| | | | | IN | 4370N2015 | A | 16 October 2015 |
| | | | | KR | 20130048270 | A | 09 May 2013 |
| | | | | US | 2011098499 | A1 | 28 April 2011 |
| | | | | US | 8415495 | B2 | 09 April 2013 |
| | | | | KR | 20100127778 | A | 06 December 2010 |
| | | | | KR | 101374950 | B1 | 14 March 2014 |
| | | | | JP | 2011511791 | A | 14 April 2011 |
| | | | | JP | 5921809 | B2 | 24 May 2016 |
| | | | | US | 7851645 | B2 | 14 December 2010 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011040117 A1
- WO 2010063780 A1
- CN 101249452 A
- CN 101121147 A
- WO 2001056971 A1
- US 2005080287 A1
- US 6207850 B1
- WO 2000073256 A1
- CN 1137788 A
- CN 103864973 A
- CN 103709278 A
- CN 104788592 A